# EUROPEAN PATENT SPECIFICATION

(11) **EP 1 173 408 B1**
(45) Date of publication and mention of the grant of the patent: **20.07.2005**
(21) Application number: 00922297.7
(22) Date of filing: 18.04.2000
(51) Int. Cl.: C07C 237/30, C07C 237/32, C07B 61/00, C07C 323/30, A61K 31/195, A61P 3/00

(54) **PPAR-(GAMMA) AGONISTS AS AGENTS FOR THE TREATMENT OF TYPE II DIABETES**
PPAR-(GAMMA) AGONISTEN ZUR BEHANDLUNG VON TYPE II DIABETES
AGONISTES PPAR-(GAMMA) EN TANT QU'AGENTS DE TRAITEMENT DU DIABETE DE TYPE II

(30) Priority: 19.04.1999 US 130011 P; 11.01.2000 US 175528 P
(43) Date of publication of application: 23.01.2002
(73) Proprietor: Coelacanth Corporation, New Brunswick, NJ 08901 (US)
(72) Inventor: KOLB, Hartmuth, C., Monmouth Junction, NJ 08852 (US); MCGEEHAN, Gerard, Chester Springs, PA 13425 (US); SHI, Zhi-Cai, North Brunswick, NJ 08902 (US); KOLLA, Laxma, Reddy, North Brunswick, NJ 08902 (US); CAVALLARO, Cullen, Allentown, NJ 08501 (US)
(74) Representative: Vossius, Volker, Dr.
(86) International application number: PCT/US2000/010416
(87) International publication number: WO 2000/063161

(56) References cited:
- WO-A-94/22834
- WO-A-94/22835
- WO-A-97/10813
- WO-A-97/31907
- WO-A-98/05331
- WO-A-98/58902
- WO-A-99/35163
- WO-A-99/37618
- WO-A-99/43642
- WO-A-99/67230
- MATSOUKAS, JOHN M. ET AL: "Differences in Backbone Structure between Angiotensin II Agonists and Type I Antagonists" J. MED. CHEM., vol. 38, no. 23, 1995, pages 4660-4669, XP002144648
- LOWARY, TODD ET AL: "Novel Type of Rigid C-Linked Glycosylacetylene-Phenylalanine Building Blocks for Combinatorial Synthesis of C-linked Glycopeptides" J. ORG. CHEM., vol. 63, no. 26, 1998, pages 9657-9668, XP002144649
- PATENT ABSTRACTS OF JAPAN vol. 018, no. 528 (C-1258), 6 October 1994 (1994-10-06) & JP 06 184086 A (ONO PHARMACEUT CO LTD), 5 July 1994 (1994-07-05)
- PATENT ABSTRACTS OF JAPAN vol. 1997, no. 07, 31 July 1997 (1997-07-31) & JP 09 087291 A (WAKAMOTO PHARMACEUT CO LTD), 31 March 1997 (1997-03-31)

## Description

This application claims the benefit of U.S. Provisional Application No. 60/130,011, filed April 19, 1999 and U.S. Provisional Application No. 60/175,528, filed January 11,2000.

### Field of the Invention

This invention relates to certain 1,4-disubstituted phenyl derivatives that act as agonists to the PPAR-γ receptor. This invention also relates to pharmaceutical compositions comprising such compounds. It further relates to the use of such compounds in treating Type II diabetes, or NIDDM. The interaction of certain 1,4-disubstituted phenyl derivatives of the invention with the nuclear receptor PPAR-γ is described. This interaction results in the pharmacological activities of these compounds.

### Description of the Related Art

Type II diabetes, or non-insulin-dependent diabetes mellitus (NIDDM), is a common metabolic disorder which has no effective treatment. NIDDM occurs predominantly in adults and involves a subnormal or inadequate amount of circulating endogenous insulin. The two defects associated with NIDDM are tissue insensitivity to insulin and impaired pancreatic B-cell response to glucose. Both of these defects are further aggravated by increased hyperglycemia, and therefore many therapeutic maneuvers seek to reduce this condition.

The currently marketed oral agents for Type II diabetes fall into several classes (i) sulphonylureas (ii) biguanides, or (iii) thiazolidinedione (TZD) derivatives such as the recently approved insulin sensitizer Rezulin™, an agonist of the PPAR-γ receptor. The sulfonyureas are an older class of drug which suffer serious drawbacks such as profound hypoglycemia and cardiovascular disease. At least three mechanisms of sulfonylurea action have been proposed: (1) release of insulin from B-cells, (2) reduction of serum glucagon levels, and (3) an extrapancreatic effect to potentiate the action of insulin on its targets. Examples of sulfonylureas are tolbutamide, tolazimide, acetohexamide, chloropropamide and second generation hyperglycemic agents such as glyburide, glipizide and glimepiride.

Biguanides, such as metformin, have also been around since the mid-1950s and are generally considered as anti-hyperglycemic agents with marginal effects on insulin responsiveness. Currently proposed mechanisms of action for biguanides include (1) direct stimulation of glycolysis in tissues, with increased glucose removal from blood; (2) reduced hepatic gluconeogenesis; (3) slowing of glucose absorption from the gastrointestinal tract; and (4) reduction of plasma glucagon levels. While biguanides do not cause hyperglycemia, there is a clear need for more effective drugs that provide glycemic control *and* promote insulin responsiveness.

TZD derivatives are a new class of oral antidiabetic drugs in which the primary mechanism appears to be increased target tissue sensitivity to insulin. Specifically, the TZD involves binding to nuclear receptors (PPAR) that regulate the transcription of a number of insulin responsive genes critical for the control of glucose and lipid metabolism. This type of drug potentiates the action of insulin to increase glucose uptake and glucose oxidation in both muscle and adipose tissue, while reducing hepatic glucose output as well as lipid synthesis in muscle and fat cells. TZDs such as troglitazone (Rezulin™), ciglitazone, englitazone, rosiglitazone and pioglitazone are said to reduce hyperglycemia, hyperinsulinemia and hypertriglyceridemia in animal models.

The recently marketed TZD, Rezulin™, while effective, has a number of post-marketing safety problems including induction of liver enzymes (e.g. P450 3A4) and hepatotoxicity with significantly associated lethality. A newer TZD, rosiglitazone, suffers a poor pharmacokinetic profile in humans, which could limit its effectiveness in a larger population. Therefore, there is a clear need for more effective novel structures of PPAR-γ agonists that provide glycemic control and promote insulin responsiveness.

The documents WO 97/31907 and WO 98/05331 disclose compounds which are stated to be PPAR agonists.

The documents WO 98/58902, JP-A-9087291 and WO 94/22835 disclose compounds similar to the compounds claimed.

These compounds are indicated to inhibit the α₄β₁ mediated adhesion to VCAM or CS-1, to inhibit angiotensin converting enzyme, and to inhibit cell adhesion respectively.

### SUMMARY OF THE INVENTION

This invention provides novel compounds of Formula I that interact with the nuclear receptor PPAR-γ.

The invention provides pharmaceutical compositions comprising the compounds of Formula I. The invention also provides compounds useful in the treatment of Type II diabetes, or NIDDM. Accordingly, a broad embodiment of the invention is directed to compounds of general Formula I: 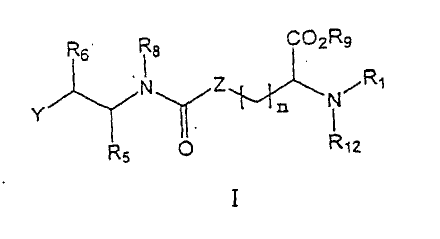 wherein:
- Z: is a 5 or 6 membered aryl or heteroaryl ring optionally substituted with up to three groups selected from C₁-C₆ alkyl, halogen or C₁-C₆ alkoxy;
- n: is 1 or 2;
- R₁ and R₁₂: are the same or different and represent hydrogen, C₁-C₆ alkyl, R₁₀SO₂, or
cycloalkyl optionally substituted with one, two, three or four groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl, or
aryl, heteroaryl, arylalkyl, or heteroarylalkyl, where the ring portion of each is optionally substituted with one, two, three or four groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl;
- R₁₀: is hydrogen or C₁-C₆ alkyl, or aryl, heteroaryl, arylalkyl or heteroarylalkyl, where the ring portion of each is optionally substituted with one, two or three groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl;
- R₉: is H or C₁-C₆ alkyl;
- Y: is hydrogen, NR₁R₁₂, OR₁, CH₂R₁, SR₁, SOR₁ or SO₂R₁; and
- R₅, R₆ and R₈,: are the same or different and represent hydrogen, C₁-C₆ alkyl, R₁₀C=O, R₁₀SO₂, or
cycloalkyl optionally substituted with one, two, three or four groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl, or
aryl, heteroaryl, arylalkyl, or heteroarylalkyl, where the ring portion of each is optionally substituted with one, two, three or four groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl; or
- R₅ and R₆: together with the carbon atom to which they are attached form a 5, 6, or 7 membered carbocyclic ring up to two of which members are optionally hetero atoms selected from oxygen, sulfur and nitrogen.

These compounds are highly selective agonists for the PPAR-γ receptor or prodrugs of agonists for the PPAR-γ receptor. These compounds are therefore useful in the treatment of Type II diabetes.

### DETAILED DESCRIPTION OF THE INVENTION

The novel compounds encompassed by the instant invention can be described by the general Formula I set forth above or the pharmaceutically acceptable non-toxic salts thereof.

In addition, the present invention also encompasses compounds of Formula II 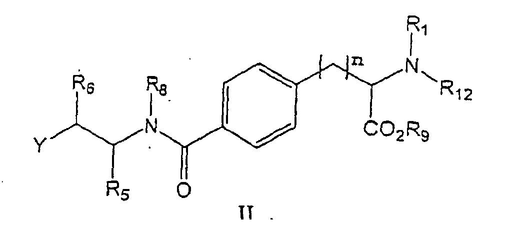 wherein n, Y, R₁ R₅, R₆, R₈, R₉ and R₁₂ are as defined above for Formula I.

Preferred compounds of Formula II are where n is 1; Y is -S-aryl, -O-aryl or -CH₂-aryl; R₉ is hydrogen or C₁-C₆ alkyl; R₈ is hydrogen or C₁-C₆ alkyl; R₁₂ is hydrogen; and R₁ is a substituted or unsubstituted aryl or arylalkyl.

By "alkyl", and "C₁-C₆ alkyl" in the present invention is meant straight or branched chain alkyl groups having 1-6 carbon atoms, such as, methyl, ethyl, propyl, isopropyl, n-butyl, sec-butyl, tert-butyl, pentyl, 2-pentyl, isopentyl, neopentyl, hexyl, 2-hexyl, 3-hexyl, and 3-methylpentyl. These groups may be substituted with up to four groups mentioned below for substituted aryl.

By "alkoxy", and "C₁-C₆ alkoxy" in the present invention is meant straight or branched chain alkoxy groups having 1-6 carbon atoms, such as, for example, methoxy, ethoxy, propoxy, isopropoxy, n-butoxy, sec-butoxy, tert-butoxy, pentoxy, 2-pentyl, isopentoxy, neopentoxy, hexoxy, 2-hexoxy, 3-hexoxy, and 3-methylpentoxy. These groups may be substituted with up to four groups mentioned below for substituted aryl.

By the term "halogen" in the present invention is meant fluorine, bromine, chlorine, and iodine.

A "carbocyclic group" or "cycloalkyl" is a nonaromatic cyclic ring or fused rings having from 3 to 7 ring members. Examples include cyclopropyl, cyclobutyl, and cycloheptyl. These rings may be substituted with one or more of the substituent groups mentioned below for aryl, for example alkyl, halo, amino, hydroxy, and alkoxy. Typical substituted carbocyclic groups include 2-chlorocyclopropyl, 2,3-diethoxycyclopentyl, and 2,2,4,4-tetrafluorocyclohexyl. The carbocyclic group may contain one or two heteroatoms selected from oxygen, sulfur, and nitrogen, and such ring systems may be referred to as "heterocyclyl" or "heterocyclic". Examples include pyranyl, tetrahydrofuranyl, and dioxanyl. These heterocyclyl groups may be substituted with up to four of the substituent groups mentioned for aryl to give groups such as 3-chloro-2-dioxanyl, and 3,5-dihydroxymorpholino.

By heteroaryl is meant one or more aromatic ring systems of 5-, 6-, or 7-membered rings containing at least one and up to four heteroatoms selected from nitrogen, oxygen, or sulfur. Such heteroaryl groups include, for example, thienyl, furanyl, thiazolyl, imidazolyl, (is)oxazolyl, pyridyl, pyrimidinyl, (iso)quinolinyl, napthyridinyl, benzimidazolyl, benzoxazolyl. The heteroaryl group is optionally substituted with up to four groups mentioned below for substituted aryl.

By aryl is meant an aromatic carbocyclic group having a single ring (e.g., phenyl), multiple rings (e.g., biphenyl), or multiple condensed rings in which at least one is aromatic, (e.g., 1,2,3,4-tetrahydronaphthyl, naphthyl, anthryl, or phenanthryl), which is optionally mono-, di-, or trisubstituted with, e.g., halogen, -OH, -SH, C₁-C₆ alkyl, C₁-C₆ alkoxy, C₁-C₆ alkylthio, trifluoromethyl, trifluoromethoxy, C₁-C₆ acyloxy, aryl, heteroaryl, amino, mono- or kialkylamino, and nitro. A preferred aryl is phenyl.

In certain situations, compounds of Formula I and Formula II may contain one or more asymmetric carbon atoms, so that the compounds can exist in different stereoisomeric forms. These compounds can be, for example, racemates or optically active forms. In these situations, the single enantiomers, i.e., optically active forms, can be obtained by asymmetric synthesis or by resolution of the racemates. Resolution of the racemates can be accomplished, for example, by conventional methods such as crystallization in the presence of a resolving agent, or chromatography, using, for example a chiral HPLC column.

Representative compounds of the invention are shown below in Table 1.

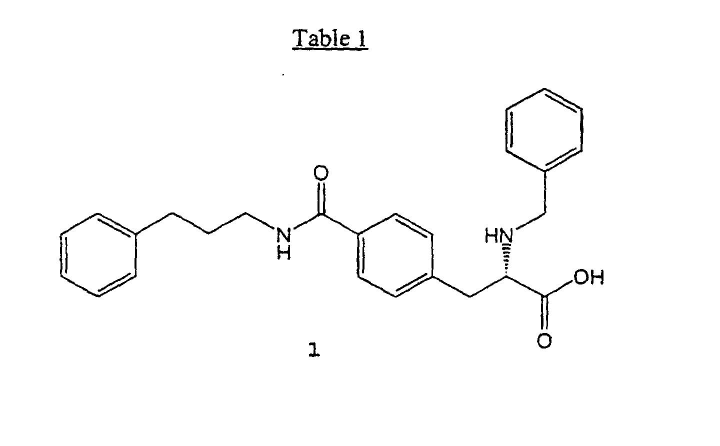

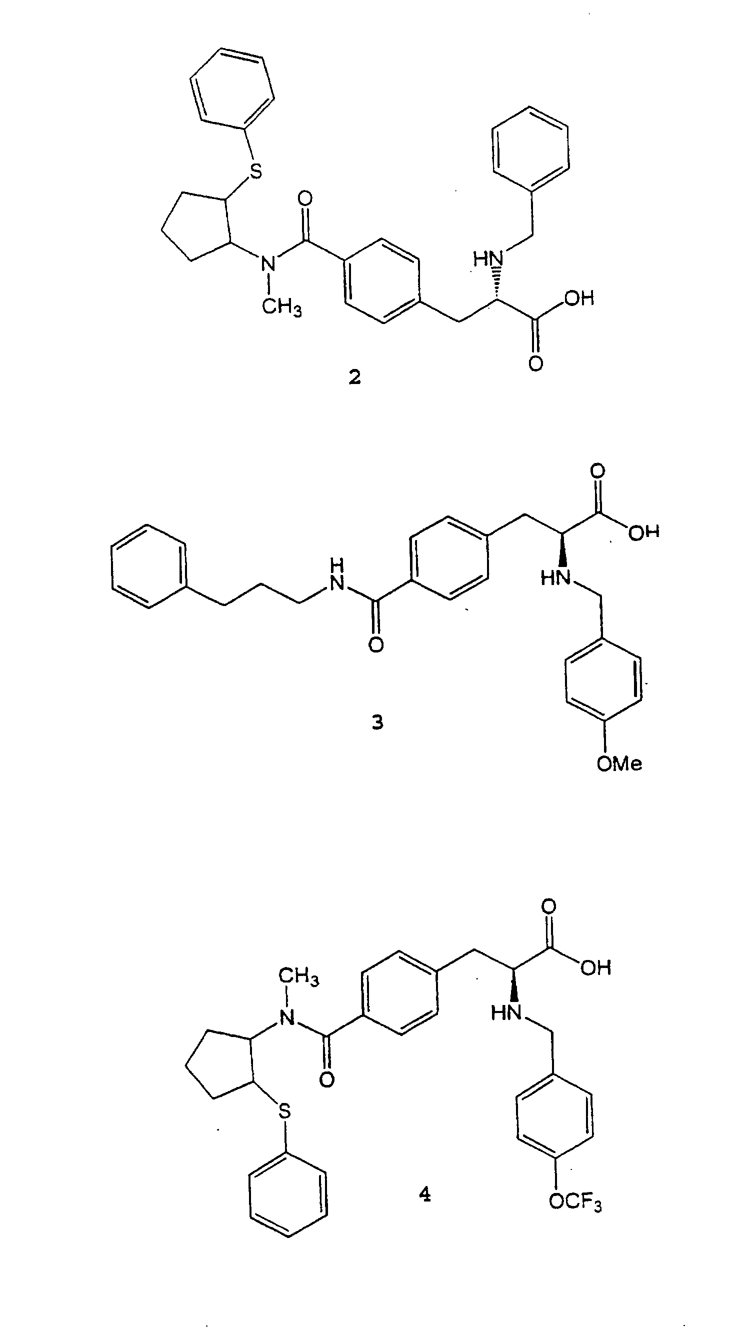

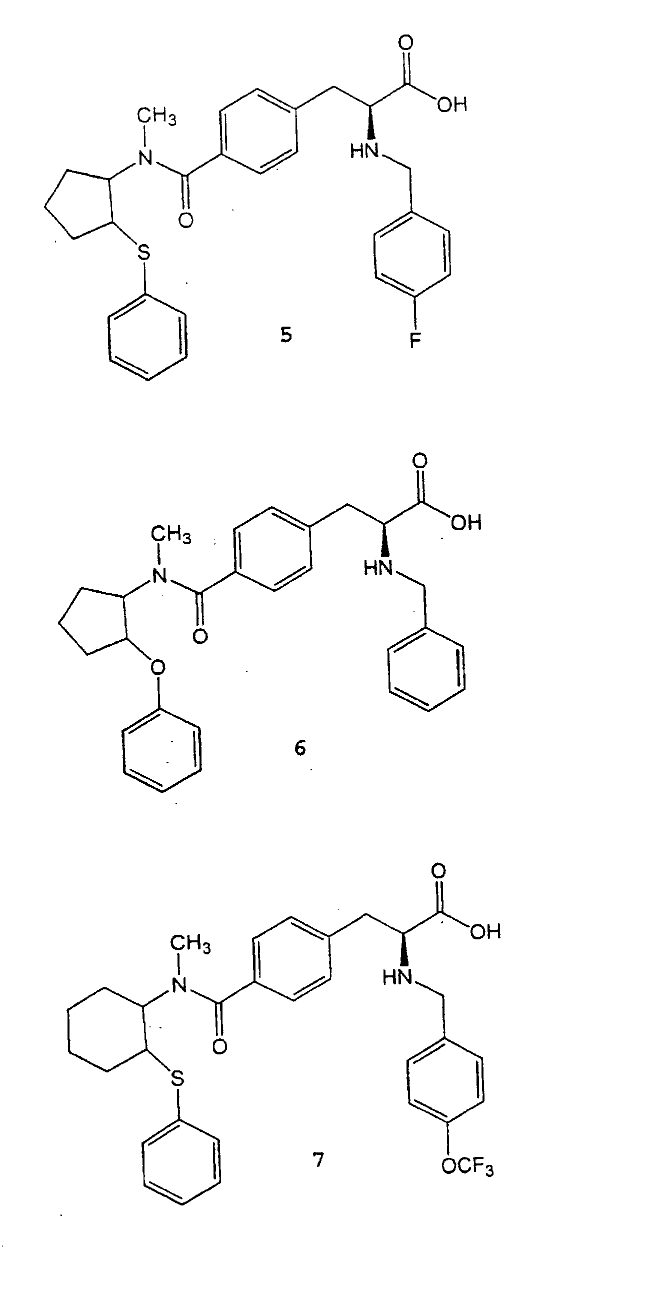

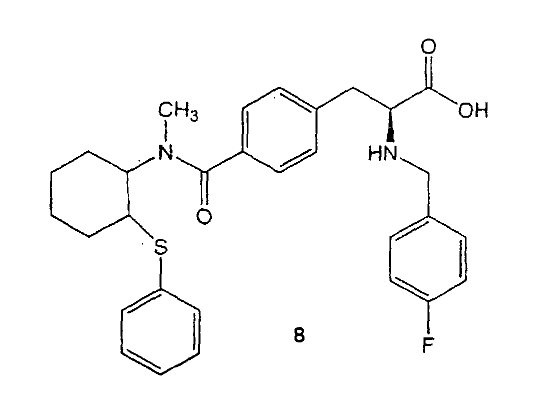

Representative compounds of the present invention, which are encompassed by Formula I, include, but are not limited to the compounds in Table I and their pharmaceutically acceptable salts. In addition, if the compound of the invention is obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds.

Non-toxic pharmaceutically acceptable salts include salts of acids such as hydrochloric, phosphoric, hydrobromic, sutfuric, sulfinic, formic, toluenesulfonic, methanesulfonic, nitric, benzoic, citric, tartaric, maleic, hydriodic, alkanoic such as acetic, HOO-(CH₂)ₙ-CO₂H where n is 0-4. Those skilled in the art will recognize a wide variety of non-toxic pharmaceutically acceptable addition salts.

The present invention also encompasses the acylated prodrugs of the compounds of Formula I. Those skilled in the art will recognize various synthetic methodologies which may be employed to prepare non-toxic pharmaceutically acceptable addition salts and acylated prodrugs of the compounds encompassed by Formula I.

The compounds of Formula I and their salts are suitable for use in the treatment of Type II diabetes, both in human and non-human animals and domestic pets or companion animals, especially dogs and cats and farm animals such as sheep, swine and cattle.

The compounds of general Formula I may be administered orally, topically, parenterally, by inhalation or spray, or rectally in unit dosage formulations containing conventional non-toxic pharmaceutically acceptable carriers, adjuvants, and vehicles. The term parenteral as used herein includes subcutaneous injections, intravenous, intramuscular, intrasternal injection or infusion techniques. In addition there is provided a pharmaceutical formulation comprising a compound of general Formula I and a pharmaceutically acceptable carrier. One or more compounds of general Formula I may be present in association with one or more non-toxic pharmaceutically acceptable carriers and/or diluents and/or adjuvants and if desired other active ingredients. The pharmaceutical compositions containing compounds of general Formula I may be in a form suitable for oral use, for example, as tablets, troches, lozenges, aqueous or oily suspensions, dispersible powders or granules, emulsions, hard or soft capsules, or syrups or elixirs.

Compositions intended for oral use may be prepared by any method known to the art for the manufacture of pharmaceutical compositions and such compositions may contain one or more agents selected from the group consisting of sweetening agents, flavoring agents, coloring agents, and preserving agents in order to provide pharmaceutically elegant and palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, cornstarch or align acid; binding agents, for example starch, gelatin, or acacia; and lubricating agents, for example, magnesium stearate, stearic acid, or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distaerate may be employed.

Formulations for oral use may also be presented as hard gelatin capsules wherein the active ingredient is mixed with an inert solid diluent, for example, calcium carbonate, calcium phosphate, or kaolin, or as soft gelatin capsules wherein the active ingredient is mixed with water or an oil medium, for example peanut oil, liquid paraffin, or olive oil.

Aqueous suspensions contain the active materials with excipients suitable for the manufacture of aqueous suspensions. Such excipients are suspending agents, for example, sodium cerboxymethylcellulose, methylcellulose, hydropropylmethylcellulose, sodium alginate, polyvinylpyrrolidone, gum tragacanth, and gum acacia; dispersing or wetting agents may be a naturally occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitanmonooleate. The aqueous suspensions may also contain one or more preservatives, for example, ethyl or n-propyl p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil, or coconut oil, or in a mineral oil such as liquid paraffin. The oily suspensions may contain a thickening agent, for example beeswax, hard paraffin, or cetyl alcohol. Sweetening agents such as those set forth above, and flavoring agents may be added to provide palatable oral preparations. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent, and one or more preservatives. Suitable dispersing or wetting agents and suspending agents are exemplified by those already mentioned above. Additional excipients, for example sweetening, flavoring, and coloring agents, may also be present.

Pharmaceutical compositions of the invention may also be in the form of oil-in-water emulsions. The oily phase may be a vegetable oil, for example olive oil or arachis oil, or a mineral oil, for example liquid paraffin, or mixtures of these. Suitable emulsifying agents may be naturally occurring gums, for example gum acacia or gum tragacanth, naturally occurring phosphatides, for example soy bean, lecithin, and esters or partial esters derived from fatty acids and hexitol, anhydrides, for example sorbitan monoleate, and condensation products of the said partial esters with ethylene oxide, for example polyoxyethylene sorbitan momoleate. The emulsions may also contain sweetening and flavoring agents.

Syrups and elixirs may be formulated with sweetening agents, for example glycerol, propylene glycol, sorbitol, or sucrose. Such formulations may also contain a demulcent, a preservative, and flavoring and coloring agents. The pharmaceutical compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parentally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono or digylcerides. In addition, fatty acids such as oleic acid find use in the preparation of injecatables.

The compounds of general Formula I may also be administered in the form of suppositories for rectal administration of the drug. These compositions can be prepared by mixing the drug with a suitable non-irritating excipient which is solid at ordinary temperatures but liquid at rectal temperature and will therefore melt in the rectum to release the drug. Such materials are cocoa butter and polyethylene glycols.

Compounds of general Formula I may be administered parenterally in a sterile medium. The drug, depending on the vehicle and concentration used, can either be suspended or dissolved in the vehicle. Advantageously, adjuvants such as local anesthetics, preservatives, and buffering agents can be dissolved in the vehicle.

It will be understood that the specific dose level for any particular patient will depend upon a variety of factors including the activity of the specific compound employed, the patient's age, body weight, general health, sex, and diet, and the time of administration, route of administration, rate of excretion, drug combination, and the severity of the particular disease undergoing therapy.

For administration to non-human animals, the composition may also be added to the animal feed or drinking water. It will be convenient to formulate these animal feed and drinking water compositions with a mullet-dose of the drug so that the animal takes in an appropriate quantity of the composition along with its diet. It will also be convenient to present the composition as a premix for addition to the feed or drinking water.

An illustration of the preparation of compounds of the present invention is given in Scheme I and Scheme II. In Scheme II, the groups R₅, R₆, R₈ and Y are as defined in general Formula I; and
- R₃: is hydrogen, C₁-C₆ alkyl, or
aryl, heteroaryl, arylalkyl, or heteroarylalkyl, where the ring portion of each is optionally substituted with one, two, three or four groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl, or
- R₃: together with the carbon atom to which it is attached forms an optionally substituted cycloalkyl.

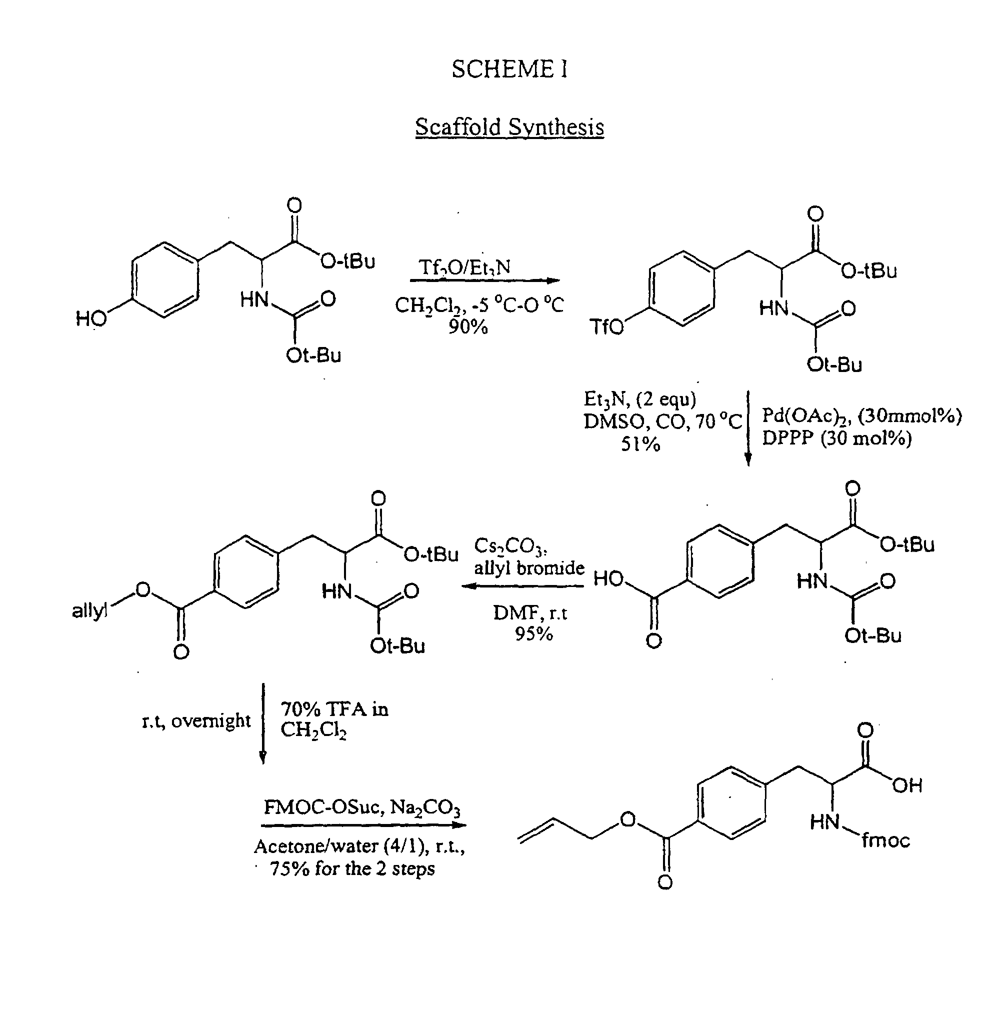

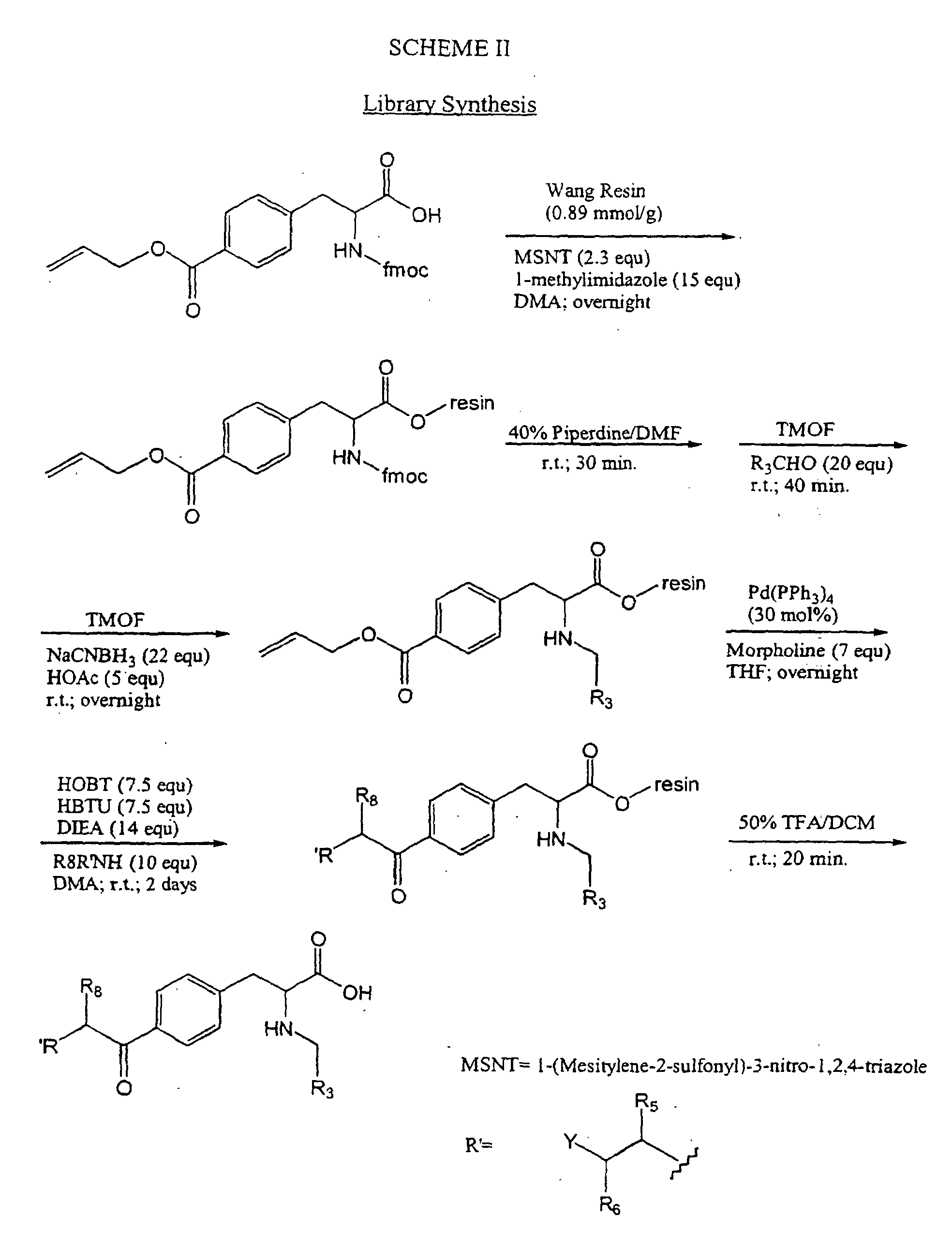

Those having skill in the art will recognize that the starting materials may be varied and additional steps employed to produce compounds encompassed by the present invention, as demonstrated by the following examples.

The invention is illustrated further by the following examples which are not to be construed as limiting the invention in scope or spirit to the specific procedures described in them.

The starting materials and various intermediates may be obtained from commercial sources, prepared from commercially available organic compounds, or prepared using well known synthetic methods.

Representative examples of methods for preparing intermediates of the invention are set forth below.

### Example 1

### 1. Loading scaffold on Wang Resin

6.0 g of Wang Resin (0.89 mmol/g, 5.34 mmol) is washed with DMA (N,N-dimethylacetamide) 3 x 40 ml, DCM (dichloromethane) 3 x 40 ml in the peptide vessel. The resin is then dried over a vacuum pump for 2 hrs.

The treated resin is swelled in 70 ml of DCM. 5.83 g of Fmoc-amino acid (12.38 mmol), 3.8 g of 1-(mesitylene-2-sulfonyl)-3-nitro-1,2,4-triazole (MSNT) (12.8 mmol) and 32 ml of 1-methylimidazole (402 mmol) are added subsequently. The resultant mixture is shaken on the orbital shaker (200 rpm) for 3.5 hrs. The resin is drained and washed with DMA (4 x 50 ml), tetrahydrofuran (THF) (4 x 50ml), DCM (4 x 50 ml) and dried under vacuum pump overnight.

2 ml of DCM and 2 ml of TFA are added to 237 mg of coupled resin in peptide vessel. The mixture is shaken at room temperature for 30 min. The resin is filtered off and washed with DCM/trifluoroacetic acid (TFA) =1/1 (3 x 2 ml), DCM (3 x 5 ml). The filtrates are combined together. Removal of solvents gives a white solid (70 mg). After the blank of resin is deducted (~6mg), the loading efficiency of coupling reaction between Fmoc protected amino acid made and Wang resin is 0.573 mmol/g.

### 2. Deprotection of Fmoc group

Dimethylformamide (DMF) (60 ml) and piperidine (40 ml) are added to Fmoc-(4-allyloxycarbonyl)-L-Tyr-Wang resin (6g, 3.44 mmol). The mixture is shaken (200 rpm) at room temperature for 40 min. The resin is then drained and washed with DMF (3 x 40 ml), THF (3 x 40 ml), DCM (3 x 40 ml) and dried under high vacuum pump overnight.

### 3. Reductive Amination

The above amine resin is swelled in 60 ml of TMOF, and benzaldehyde (9.6 ml, 94.4 mmol) is added and the mixture is shaken for 40 min. The resin is drained and washed with TMOF (2 x 40 ml) to remove excess aldehyde. The resin is re-swelled in 60 ml of TMOF and sodium cyanoborohydride (5.6 g, 89.2 mmol) is added followed by 1 ml of acetic acid. The mixture is shaken (200 rpm) at room temperature overnight. The resin is then drained and washed with MeOH (3 x 40 ml), DMF(3 x 40 ml), THF (3 x 40 ml), DCM (3 x 40 ml) and dried under high vacuum pump overnight.

### 4. Allyl deprotection

The above resin is swelled in 60 ml of anhydrous THF and 2.1 ml of morpholine (24.2 mmol, 7 eq.) is added followed by 1.2 g of Pd(PPh₃)₄ (30% mol). The mixture is shaken (200 rpm) at room temperature overnight. The resin is then drained and washed with HOAc (3 x 40 ml), DMF (3 x 40 ml), THF (3 x 40 ml), DCM (3 x 40 ml) and dried under a high vacuum pump

### 5. Coupling with 3-phenyl-1-propylamine

The above resin is swelled in 70 ml of DMA. HOBt (3.48 g, 25.8 mmol, 7.5 eq.), HBTU (9.78 g, 25.8 mmol, 7.5 eq.), DIEA (8.37 ml, 14 eq.) and 3-phenyl-1-propylamine (4.9 ml, 10 eq.) are added. The mixture is shaken (200 rpm) at room temperature for 2 days. The resin is then drained and washed with DMF (4 x 40 ml), THF (4 x 40 ml) and then DCM (4 x 40 ml) and dried under high vacuum pump.

### 6. Cleavage from resin

The above resin is swelled in 60 ml of DCM and 40 ml of TFA is added. The mixture is shaken at room temperature for 30 min. The resin is drained and the TFA/DCM solution is collected. The resin is washed with DCM (2 x 30 ml) and all organic solutions are combined. Removal of solvents gives crude product, which is purified by column chromatography (SiO₂, CHCl₃/MeOH = 10/1 to 4/1) to produce 811 mg of a white solid.

### Example 2

The following compounds are prepared essentially as described in example 1:
(a) (2S)-2-[benzylamino]-3-{4-[N-(3-phenylpropyl)carbamoyl]phenyl} propanoic acid (**Compound 1**);
(b) (2S)-3-{4-[N-methyl-N(2-phenylthiocyclopentyl)carbamoyl]phenyl}-2-[benzylamino]propanoic acid (**Compound 2**);
(c) (2S)-2-{[(4-methoxyphenyl)methyl)amino}-3-{4-[N-(3-phenylpropyl) carbamoyl]phenyl}propanoic acid (**Compound 3**);
(d) (2S)-3- {4-[N-methyl-N-(2-phenylthiocyclopentyl)carbamoyl]phenyl}-2-({[4-(trifluoromethoxy)phenyl]methyl}amino)propanoic acid (**Compound 4**);
(e) (2S)-2-{[(4-fluorophenyl)methyl]amino}-3-{4-[N-methyl-N-(2-phenylthiocyclopentyl)carbamoyl]phenyl}propanoic acid (**Compound 5**);
(f) (2S)-3- {4-[N-methyl-N-(2-phenoxycyclopentyl)carbamoyl]phenyl}-2-[benzylamino]propanoic acid (**Compound 6**);
(g) (2S)-3-{4-[N-methyl-N-(2-phenylthiocyclohexyl)carbamoyl]phenyl}-2-({[4-(trifluoromethoxy)phenyl]methyl}amino)propanoic acid (**Compound 7**); and
(h) (2S)-2-{[(4-fluorophenyl)methyl]amino}-3-{4-[N-methyl-N-(2-phenylthiocyclohexyl)carbamoyl]phenyl}propanoic acid (**Compound 8**).

### Example 3

### Human Adipocyte Differentiation

Assays are carried out on primary human subcutaneous preadipocytes. Preadipocytes are inoculated at high density in preadipocyte medium (DME/F-10, 1:1, (v/v) containing 10% fetal calf serum) and maintained overnight for attachment. Drug treatment is initiated the second day by changing to serum free medium containing test compounds. The basal medium, which is used as the negative control, contains DME/F-10, biotin (33 µM), pantothenate (17 µM), insulin (100nM), and dexamethasone (1µM) and BRL 49653 is included as a positive control. The culture is maintained for 14 days with the compound treatment during the first five days. At the end of the culture, cells are fixed in 5% formalin and stained with oil red-O dye. The dye is extracted by isopropanol and quantitated by measure the optical density at 500 nm. EC50 values found in Table 1 reflect the human adipocyte differentiation of the compound in presence of insulin and dexamethasone.

**Table 1**

| Human Adipocyte Differentiation | | |
|---|---|---|
| **Compound** | **EC50 Differentiation** | **EC50 Inhibition of Differentiation** |
| 1 | 150 nm | >10 µm |
| 2 | 175 nm | >10 µm |
| Rosiglitizone | 100 nm | > 10 µm |

### Example 4

### Binding to PPAR-γ Receptor

Compounds are assayed for their ability to inhibit the binding of 3H-PPAR-γ ligand to the LBD (ligand binding domain) of the PPAR-γ receptor. Compounds are tested at 4 concentrations (1µM to 1 nM) in triplicate. Compounds are incubated with H3-PPAR ligand (8 nM final concentration) and LBD (210 ng/assay well) in assay buffer (50mM Tris pH8.0. 50mM KCL, 2mM EDTA, 0.3% CHAPS, 0.1 mg/ml BSA, 10mM DTT) for 2 hours with gentle shaking. Non-specific binding is measured in the presence of 10 µM excess cold competitor. Bound ligand is separated using gel filtration plates (Edge Biosystems 31909) and captured on 96 well Wallac MicroBeta plates (1450-5150 by centrifugation for 5 minutes at 2500rpm. Radioactivity is measured in a Wallac Micro Beta counter. Table 2 gives the IC50 values of compounds 1 and 2.

**Table 2**

| **Compound** | **IC**_{**50**} **(µm)** |
|---|---|
| 1 | 1.7 |
| 2 | 3.3 |

### Example 5

### Blood Glucose Lowering in Diabetic Rats

Male Zucker lean rats, 24-28 weeks of age, are fed high fat chow (AIN-93-M formulation) for two weeks to elevate blood glucose. At blood glucose concentrations over 160 mg/dl, a three day pre-study baseline is established by measuring blood glucose daily (at 0700-0800 hrs) via tail vein blood extraction and measurement using a glucometer. Animals are segregated into groups of 5 based on weights and baseline glucose. Compounds are then administered to the animals at various concentrations (30 mg compound/kg body weight or 10 mg compound/kg body weight). The drugs were suspended in 0.5% carboxymethylcellulose (Sigma) in a total gavage volume of 250 µL. Animals are dosed for seven days, once daily in the moming after blood glucose and weight determination. At the end of the seven-day period, the animals are euthanized by exsanguination after isofluorane anesthesia. Table 3 depicts the normalization of blood glucose in the rats.

**Table 3**

| Normalization of Blood Glucose in Zucker Obese Rats | | | |
|---|---|---|---|
| Treatment Day | Blood Glucose (mg/dl ± SEM) | | |
| | Vehicle | Cmpd. 1 | Cmpd. 2 |
| 0 | 129±7 | 133±11 | 133±11 |
| 1 | 134±15 | 134±11 | 136±5 |
| 2 | 133±7 | 105±7 | 104±1 |
| 3 | 129±5 | 105±4 | 100±9 |
| 4 | 143±3 | 97±16 | 93±6 |
| 5 | 120±10 | 113±3 | 100±4 |
| 6 | 127±5 | 105±6 | 101±5 |
| 7 | 132±6 | 104±8 | 92±6 |

The invention and manner and process of making and using it, are now described in such full, clear, concise and exact terms as to enable any person skilled in the art to which it pertains, to make and use the same. It is to be understood that the foregoing describes preferred embodiments of the present invention. To particularly point out and distinctly claim the subject matter regarded as invention, the following claims conclude this specification.

## Claims

1. A compound of the formula: 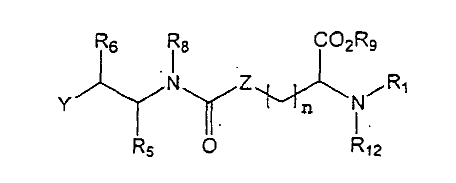 or the pharmaceutically acceptable non-toxic salts thereof wherein:
Z is a 5 or 6 membered aryl or heteroaryl ring optionally substituted with up to three groups selected from C₁-C₆ alkyl, halogen or C₁-C₆ alkoxy;
n is 1 or 2;
R₁ and R₁₂ are the same or different and represent hydrogen, C₁-C₆ alkyl, SO₂(R₁₀), or cycloalkyl optionally substituted with one, two, three or four groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl, or aryl, heteroaryl, arylalkyl, or heteroarylalkyl, where the ring portion of each is optionally substituted with one, two, three or four groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl;
R₁₀ is hydrogen or C₁-C₆ alkyl, or aryl, heteroaryl, arylalkyl or heteroarylalkyl, where the ring portion of each is optionally substituted with one, two or three groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl;
R₉ is H or C₁-C₆ alkyl;
Y is hydrogen, NR₁R₁₂, OR₁, CH₂R₁, SR₁, SOR₁ or SO₂R₁; and
R₅, R₆ and R₈, are the same or different and represent hydrogen, C₁-C₆ alkyl, R₁₀C=O, R₁₀SO₂, or
cycloalkyl optionally substituted with one, two, three or four groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl, or
aryl, heteroaryl, arylalkyl, or heteroarylalkyl, where the ring portion of each is optionally substituted with one, two, three or four groups independently selected from halogen, trifluoromethyl, trifluoromethoxy, cyano, nitro, carboxyl, alkoxycarboxy, alkylcarboxy, hydroxy, C₁-C₆ alkyl, C₁-C₆ alkoxy, amino, or mono or dialkylamino where each alkyl portion is C₁-C₆ alkyl; or
R₅ and R₆ together with the carbon atom to which they are attached form a 5, 6, or 7 membered carbocyclic ring up to two of which members are optionally hetero atoms selected from oxygen, sulfur and nitrogen.

2. A compound of claim 1 having the formula:

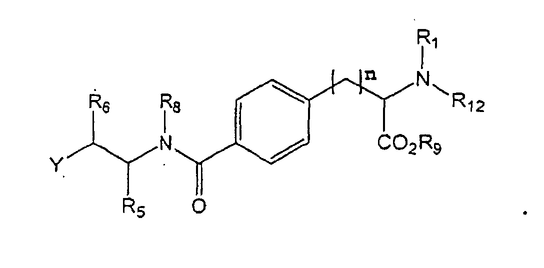

3. A compound according to claim 2 wherein n is 1.

4. A compound according to claim 2 wherein R₈ is selected from hydrogen or C₁-C₆ alkyl.

5. A compound according to claim 1 which is selected from
(2S)-2-[benzylamino]-3-{4-[N-(3-phenylpropyl)carbamoyl]phenyl} propanoic acid;
(2S)-3-{4-[N-methyl-N(2-phenylthiocyclopentyl)carbamoyl]phenyl}-2-[benzylamino]propanoic acid;
(2S)-2-{[(4-methoxyphenyl)methyl]amino}-3-{4-[N-(3-phenylpropyl) carbamoyl]phenyl}propanoic acid;
(2S)-3-{4-[N-methyl-N-(2-phenylthiocyclopentyl)carbamoyl]phenyl}-2-({[4-(trifluoromethoxy)phenyl]methyl}amino)propanoic acid;
(2S)-2-{[(4-fluorophenyl)methyl]amino}-3-{4-[N-methyl-N-(2-phenylthiocyclopentyl)carbamoyl]phenyl}propanoic acid;
(2S)-3-{4-[N-methyl-N-(2-phenoxycyclopentyl)carbamoyl]phenyl}-2-[benzylamino]propanoic acid;
(2S)-3-{4-[N-methyl-N-(2-phenylthiocyclohexyl)carbamoyl]phenyl}-2-({[4-(trifluoromethoxy)phenyl]methyl}amino)propanoic acid; and
(2S)-2-{[(4-fluorophenyl)methyl]amino}-3-[4-[N-methyl-N-(2-phenylthiocyclohexyl)carbamoyl]phenyl}propanoic acid.

6. A pharmaceutical composition comprising a compound according to Claim 1 and a pharmaceutically acceptable carrier.

7. Use of a compoud according to Claim 1 for the preparation of a medicament for treating Type II diabetes in a mammal.

## Patentansprüche

1. Verbindung der Formel: 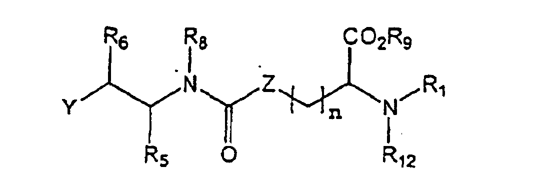 oder die pharmazeutisch verträglichen nicht toxischen Salze davon, worin:
Z ein 5- oder 6-gliedriger Aryl- oder Heteroarylring ist, der gegebenenfalls mit bis zu drei Gruppen substituiert ist, ausgewählt aus C₁-C₆-Alkylgruppe, Halogenatom oder C₁-C₆-Alkoxygruppe,
n den Wert 1 oder 2 aufweist,
R₁ und R₁₂ gleich oder unterschiedlich sind und einem Wasserstoffatom, einer C₁-C₆-Alkyl-, SO₂(R₁₀)-Gruppe oder
Cycloalkylgruppe, die gegebenenfalls mit einer, zwei, drei oder vier Gruppen substituiert ist, unabhängig voneinander ausgewählt aus Halogenatom, Trifluormethyl-, Trifluormethoxy-, Cyan-, Nitro-, Carboxyl-, Alkoxycarboxy-, Alkylcarboxy-, Hydroxy-, C₁-C₆-Alkyl-, C₁-C₆-Alkoxy-, Amino- oder Mono- oder Dialkylaminogruppe, wobei jeder Alkylanteil eine C₁-C₆-Alkylgruppe ist, oder
Aryl-, Heteroaryl-, Arylalkyl- oder Heteroarylalkylgruppe, worin der Ringanteil jeder Gruppe gegebenenfalls mit einer, zwei, drei oder vier Gruppen substituiert ist, unabhängig voneinander ausgewählt aus Halogenatom, Trifluormethyl-, Trifluormethoxy-, Cyan-, Nitro-, Carboxyl-, Allcoxycarboxy-, Alkylcarboxy-, Hydroxy-, C₁-C₆-Alkyl-, C₁-C₆-Alkoxy-, Amino- oder Mono- oder Dialkylaminogruppe, wobei jeder Alkylanteil eine C₁-C₆-Alkylgruppe ist, entsprechen,
R₁₀ ein Wasserstoffatom oder eine C₁-C₆-Alkylgruppe oder Aryl-, Heteroaryl-, Arylalkyl- oder Heteroarylalkylgruppe ist, worin der Ringanteil jeder Gruppe gegebenenfalls mit einer, zwei oder drei Gruppen substituiert ist, unabhängig voneinander ausgewählt aus Halogenatom, Trifluormethyl-, Trifluormethoxy-, Cyan-, Nitro-, Carboxyl-, Alkoxycarboxy-, Alkylcarboxy-, Hydroxy-, C₁-C₆-Alkyl-, C₁-C₆-Alkoxy-, Amino- oder Mono- oder Dialkylaminogruppe, wobei jeder Alkylanteil eine C₁-C₆-Alkylgruppe ist,
R₉ ein H-Atom oder eine C₁-C₆-Alkylgruppe ist,
Y ein Wasserstoffatom, eine NR₁R₁₂-, OR₁-, CH₂R₁-, SR₁-, SOR₁- oder SO₂R₁-Gruppe ist und
R₅, R₆ und R₈ gleich oder unterschiedlich sind und einem Wasserstoffatom, einer C₁-C₆-Alkyl-, R₁₀C=O-, R₁₀SO₂-Gruppe oder
Cycloalkylgruppe, die gegebenenfalls mit einer, zwei, drei oder vier Gruppen substituiert ist, unabhängig voneinander ausgewählt aus Halogenatom, Triffuormethyl-, Trifluormethoxy-, Cyan-, Nitro-, Carboxyl-, Alkoxycarboxy-, Alkylcarboxy-, Hydroxy-, C₁-C₆-Alkyl-, C₁-C₆-Alkoxy-, Amino- oder Mono- oder Dialkylaminogruppe, wobei jeder Alkylanteil eine C₁-C₆-Alkylgruppe ist, oder
Aryl-, Heteroaryl-, Arylalkyl- oder Heteroarylalkylgruppe, worin der Ringanteil jeder Gruppe gegebenenfalls mit einer, zwei, drei oder vier Gruppen substituiert ist, unabhängig voneinander ausgewählt aus Halogenatom, Trifluormethyl-, Trifluormethoxy-, Cyan-, Nitro-, Carboxyl-, Alkoxycarboxy-, Alkylcarboxy-, Hydroxy-, C₁-C₆-Alkyl-, C₁-C₆-Alkoxy-, Amino- oder Mono- oder Dialkylaminogruppe, wobei jeder Alkylanteil eine C₁-C₆-Alkylgruppe ist, entsprechen oder
R₅ und R₆ zusammen mit dem Kohlenstoffatom, an das sie gebunden sind, einen 5-, 6- oder 7-gliedrigen carbocyclischen Ring bilden, wobei bis zu zwei dieser Glieder gegebenenfalls Heteroatome, ausgewählt aus Sauerstoff-, Schwefel- und Stickstoffatomen, sind.

2. Verbindung nach Anspruch 1 der Formel:

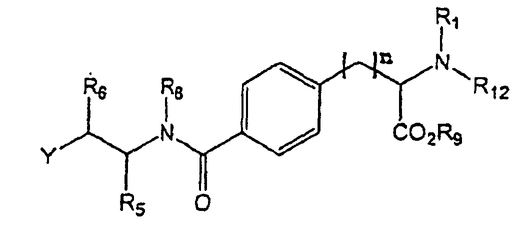

3. Verbindung gemäß Anspruch 2, wobei n den Wert 1 aufweist.

4. Verbindung gemäß Anspruch 2, wobei R₈ ausgewählt ist aus Wasserstoffatom oder C₁-C₆-Alkylgruppe.

5. Verbindung gemäß Anspruch 1, die ausgewählt ist aus
(2S)-2-[Benzylamino]-3-{4-[N-(3-phenylpropyl)carbamoyl]phenyl}propansäure,
(2S)-3-{4-[N-Methyl-N-(2-phenylthiocyclopentyl)carbamoyl]phenyl}-2-[benzylamino]propansäure,
(2S)-2-{[(4-Methoxyphenyl)methyl]amino}-3-{4-[N-(3-phenylpropyl)carbamoyl]-phenyl}propansäure,
(2S)-3-{4-[N-Methyl-N-(2-phenylthiocyclopentyl)carbamoyl]phenyl}-2-({[4-(trifluormethoxy)phenyl]methyl}amino)propansäure,
(2S)-2-{[(4-Fluorphenyl)methyl]amino}-3-{4-[N-methyl-N-(2-phenylthiocyclopentyl)carbamoyl]phenyl}propansäure,
(2S)-3-{4-[N-Methyl-N-(2-phenoxycyclopentyl)carbamoyl]phenyl}-2-[benzylamino]-propansäure,
(2S)-3-{4-[N-Methyl-N-(2-phenylthiocyclohexyl)carbamoyl]phenyl}-2-({[4-(trifluormethoxy)phenyl]methyl}amino)propansäure und
(2S)-2-{[(4-Fluorphenyl)methyl]amino}-3-{4-[N-methyl-N-(2-phenylthiocyclohexyl)-carbamoyl]phenyl}propansäure.

6. Pharmazeutische Zusammensetzung, die eine Verbindung gemäß Anspruch 1 und einen pharmazeutisch verträglichen Träger umfasst.

7. Verwendung einer Verbindung gemäß Anspruch 1 zur Herstellung eines Medikaments zum Behandeln von Typ II-Diabetes in einem Säuger.

## Revendications

1. Composé de formule: 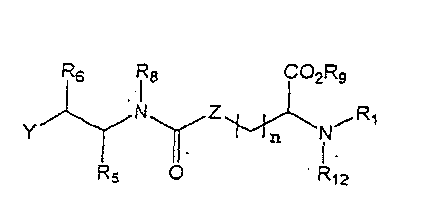 ou ses sels non-toxiques pharmaceutiquement acceptables, où:
Z est un cycle aryle ou hétéroaryle ayant 5 ou 6 chaînons, en option substitué par jusqu'à trois groupes choisis parmi un alkyle en C₁-C₆, un halogène ou un alcoxy en C₁-C₆;
n est 1 ou 2;
R₁ et R₁₂ sont identiques ou différents et représentent l'hydrogène, un alkyle en C₁-C₆, SO₂(R₁₀), ou
un cycloalkyle en option substitué par un, deux, trois ou quatre groupes choisis indépendamment parmi les suivants: halogène, trifluorométhyle, trifluorométhoxy, cyano, nitro, carboxyle, alcoxycarboxy, alkylcarboxy, hydroxy, alkyle en C₁-C₆, alcoxy en C₁-C₆, amino, ou mono- ou dialkylamino, tandis que chaque portion alkyle est un alkyle en C₁-C₆, ou
un aryle, un hétéroaryle, un arylalkyle, ou un hétéroarylalkyle, tandis que la portion cyclique de chacun est en option substituée par un, deux, trois ou quatre groupes choisis indépendamment parmi les suivants: halogène, trifluorométhyle, trifluorométhoxy, cyano, nitro, carboxyle, alcoxycarboxy, alkylcarboxy, hydroxy, alkyle en C₁-C₆, alcoxy en C₁-C₆, amino, ou mono- ou dialkylamino, tandis que chaque portion alkyle est un alkyle en C₁-C₆;
R₁₀ est l'hydrogène ou un alkyle en C₁-C₆, ou un aryle, un hétéroaryle, un arylalkyle ou un hétéroarylalkyle, tandis que la portion cyclique de chacun est en option substituée par un, deux ou trois groupes choisis indépendamment parmi les suivants: halogène, trifluorométhyle, trifluorométhoxy, cyano, nitro, carboxyle, alcoxycarboxy, alkylcarboxy, hydroxy, alkyle en C₁-C₆, alcoxy en C₁-C₆, amino, ou mono- ou dialkylamino, tandis que chaque portion alkyle est un alkyle en C₁-C₆;
R₉ est H ou un alkyle en C₁-C₆;
Y est l'hydrogène, NR₁R₁₂, OR₁, CH₂R₁, SR₁, SOR₁ ou SO₂R₁; et
R₅, R₆ et R₈ sont identiques ou différents et représentent l'hydrogène, un alkyle en C₁-C₆, R₁₀C=O, R₁₀SO₂, ou
un cycloalkyle en option substitué par un, deux, trois ou quatre groupes choisis indépendamment parmi les suivants: halogène, trifluorométhyle, trifluorométhoxy, cyano, nitro, carboxyle, alcoxycarboxy, alkylcarboxy, hydroxy, alkyle en C₁-C₆, alcoxy en C₁-C₆, amino, ou mono- ou dialkylamino, tandis que chaque portion alkyle est un alkyle en C₁-C₆, ou
un aryle, un hétéroaryle, un arylalkyle, ou un hétéroarylalkyle, tandis que la portion cyclique de chacun est en option substituée par un, deux, trois ou quatre groupes choisis indépendamment parmi les suivants: halogène, trifluorométhyle, trifluorométhoxy, cyano, nitro, carboxyle, alcoxycarboxy, alkylcarboxy, hydroxy, alkyle en C₁-C₆, alcoxy en C₁-C₆, amino, ou mono- ou dialkylamino, tandis que chaque portion alkyle est un alkyle en C₁-C₆;
ou
R₅ et R₆ conjointement avec l'atome de carbone auquel ils sont liés forment un cycle carbocyclique à 5, 6 ou 7 chaînons, dont jusqu'à deux membres sont en option des hétéroatomes choisis parmi l'oxygène, le soufre et l'azote.

2. Composé selon la revendication 4, ayant la formule:

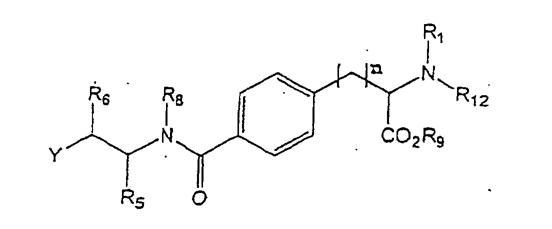

3. Composé selon la revendication 2, dans lequel n est 1.

4. Composé selon la revendication 2, dans lequel R₈ est choisi parmi l'hydrogène ou un alkyle en C₁-C₆.

5. Composé selon la revendication 1, qui est choisi parmi les suivants:
acide (2S)-2-[benzylamino]-3-{4-[N-(3-phénylpropyl)carbamoyl]-phényl}propanoïque;
acide (2S)-3-{4-[N-méthyl-N-(2-phénylthiocyclopentyl)carbamoyl]-phényl}-2-[benzylamino]propanoïque;
acide (2S)-2-{[(4-méthoxyphényl)méthyl]amino}-3-{4-[N-(3-phénylpropyl)carbamoyl]phényl}propanoïque;
acide (2S)-3-{4-[N-méthyl-N-(2-phénylthiocyclopentyl)carbamoyl]-phényl}-2-({[4-(trifluorométhoxy)phényl]méthyl}amino)propanoïque;
acide (2S)-2-{[(4-fluorophényl)méthyl]amino}-3-{4-[N-méthyl-N-(2-phénylthiocyclopentyl)carbamoyl]phényl}propanoïque;
acide (2S)-3-{4-[N-méthyl-N-(2-phénoxycyclopentyl)carbamoyl]-phényl}-2-[benzylamino]propanoïque;
acide (2S)-3-{4-[N-méthyl-N-(2-phénylthiocyclohexyl)carbamoyl]-phényl}-2-({[4-(trifluorométhoxy)phényl]méthyl}amino)propanoïque; et
acide (2S)-2-{[(4-fluorophényl)méthyl]amino}-3-{4-[N-méthyl-N-(2-phénylthiocyclohexyl)carbamoyl]phényl}propanoïque.

6. Composition pharmaceutique comprenant un composé selon la revendication 1 et un support pharmaceutiquement acceptable.

7. Utilisation d'un composé selon la revendication 1 pour la préparation d'un médicament pour le traitement du diabète de type II chez un mammifère.
